# EUROPÄISCHE PATENTSCHRIFT

(11) **EP 2 397 179 B1**
(45) Veröffentlichungstag und Bekanntmachung des Hinweises auf die Patenterteilung: **21.11.2012**
(21) Anmeldenummer: 11004992.1
(22) Anmeldetag: 18.06.2011
(51) Int. Cl.: A61M 16/08, A61M 16/00

(54) **Zusatzelement zur Hochfrequenzbeatmung**
An add on element for high frequency respiration
Un élément supplémentaire pour respiration à haute fréquence

(30) Priorität: 19.06.2010 DE 102010024397
(43) Veröffentlichungstag der Anmeldung: 21.12.2011
(73) Patentinhaber: Müller, Gerhard, 23829 Wittenborn (DE)
(72) Erfinder: Müller, Gerhard, 23829 Wittenborn (DE)

(56) Entgegenhaltungen:
- EP-A2- 1 106 197
- EP-A2- 1 108 440
- WO-A2-98/07401
- US-A- 4 681 100
- US-A1- 2010 071 688

## Beschreibung

Die Erfindung bezieht sich auf ein Zusatzelement eines Grundelementes mit einem Düsensystem zur Hochfrequenzbeatmung mit einem offenen Beatmungssystem, wie bei einem Bronchoskop, Laryngoskop, wobei über einen Anschluss mittels des Düsensystems aufbereitetes Atemgas mit hohem Druck hochfrequent mit zusätzlich angesaugter Umgebungsluft über eine Öffnung als vertikale Instrumentenzuführung einleitbar ist.

Bei bekannten Hochfrequenzbeatmungen in einem offen Beatmungssystem wird über ein Düsensystem Atemgas mit hohem Druck appliziert. Beim Austritt aus einer eingesetzten Düse expandiert das Gas und erzeugt je nach Art der Applikation einen erheblichen Geräuschpegel. Dieser Geräuschpegel ist physikalisch bedingt und ist nicht reduzierbar ohne die Effizienz der Beatmung zu reduzieren.

Bei diesem Verfahren wird beim Austritt aus einer Düsenöffnung Umgebungsluft mitgerissen, die wesentlich zur Beatmung beiträgt. Die Gesamtatemgasmenge aus expandiertem Gas und dem eingesogenen Atemgas sorgen zusammen für die notwendige Gasmenge für den Gasaustausch. Bei der Ausatmung dieser zugeführten Gasmenge werden zwangsläufig die in der Lunge und im Respirationsweg befindlichen Keime mit ausgeatmet und gefährden den Operateur und das im Behandlungsbereich befindliche Personal.

Bei länger andauernden Eingriffen kommt es bei der Verwendung medizinischer Gase, die bestimmungsgemäß kalt und sehr trocken sind, zu Belastungen und gegebenenfalls zu Schädigungen der Schleimhäute des Patienten.

Aus der US 2010/071688 A1 ist bereits eine Anordnung für ein geschlossenes Beatmungssystem mit einer Zuführung für ein einsetzbares Inhalationsgerät mit einem Verschlusselement bekannt. Hierdurch wird ein starres System geschaffen und ist nicht entsprechend auf eine Kontrolle und operative Eingriffe mit einem offenen System übertragbar, wobei eine Behandlung geschützt durchführbar ist.

Außerdem ist nach der US 4,681,100 A ein Grundelement der gattungsgemäßen Art für ein offenes System mit einer Arbeitsöffnung bekannt, das die vorgenannten Mängel aufweist.

Die Aufgabe der Erfindung ist es, eine gattungsgemässe Anordnung zur Hochfrequenzbeatmung zu verbessern, die mit einfachen Mitteln, insbesondere eine Geräuschbeeinträchtigung, ohne Beeinträchtigung der Arbeitsweise, wesentlich herabsetzt und bei Ausatmung eine Beeinträchtigung des Personals vermindert sowie eine Nachrüstung bestehender Geräte ermöglicht.

Die Lösung dieser Aufgabe erfolg erfindungsgemäß dadurch, dass die Öffnung des Grundelementes mit einem Zusatzelement verbunden ist, das eine Zuluftöffnung für Umgebungsluft und eine Abluftöffnung aufweist sowie in Verlängerung der Instrumentenzuführung eine verschliessbare Zuführöffnung im Zusatzelement für den Instrumenteneinsatz besitzt, wobei die Zuführöffnung durch eine selbst einstellende Dichtung abgeschlossen ist und als flexibles Flächenelement eine durch Schlitze als Kreuzschlitze gebildete Einstecköffnung zur Umfassung einsetzbarer Instrumente mit unterschiedlichen Durchmessern aufweist sowie mehrere Lagen einzelner Flächenelemente der Dichtung parallel angeordnet und die gebildeten Schlitze lagenweise gegeneinander versetzt angeordnet sind.

Hierbei wird es ermöglicht, die beachtliche Geräuschbeeinträchtigung über die Dichtung wesentlich herabzusetzen und eine kontrollierte Zuführung über die Zuluftöffnung zu gewährleisten. Ferner kann über die Abluftöffnung die Atemluft kontrolliert abgeführt werden.

Ferner wird vorgeschlagen, dass die lagenweise angeordneten Flächenelemente der Dichtung aus Werkstoffen unterschiedlicher Shorehärte gebildet sind.

Zur Verbindung mit Zusatzeinheiten ist vorgesehen, dass das Zusatzelement mit seiner Zuführung die zugeordnete Zuluftöffnung für die Umgebungsluft und die Abluftöffnung für die Abluft als Anschlusstutzen aufweist.

Eine vorteilhafte Ausbildung zur Anpassung der zugeführten Atemgase besteht darin, dass die Zuluftöffnung vorgeschaltete Zusatzeinheiten zur Luftanfeuchtung und Temperierung aufweist.

Um einen Versatz der einzelnen Flächenelemente eines Dichtungssatzes zu erleichtern, wird vorgeschlagen, dass die einsetzbaren Flächenelemente der Dichtung als Dichtungssatz gegeneinander versetzte Kerben aufweisen, die einen Versatz der Schlitze definieren und die Kerben mit einem Führungssteg der Zuführöffnung korrespondieren.

Eine vorteilhafte Ausbildung besteht darin, dass das Zusatzelement als aufsetzbarer Adapter ausgebildet ist.

Alternativ wird vorgeschlagenn, dass Grundelement und Zusatzelement eine Einheit bilden.

In der Zeichnung ist ein Ausführungsbeispiel einer Vorrichtung schematisch dargestellt. Es zeigen:

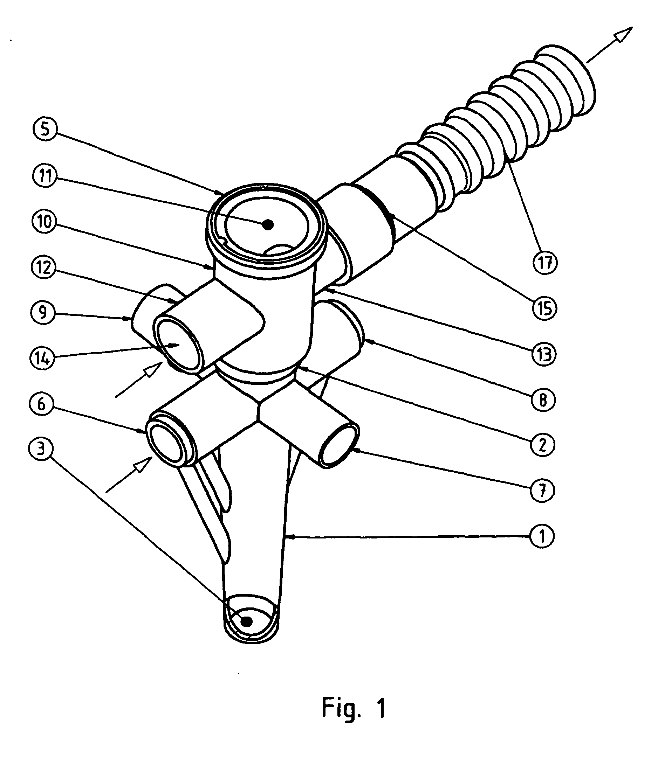

- Fig. 1: eine Prinzipdarstellung eines Bronchoskopes als Grundelement mit einem Zusatzelement als Adapter,

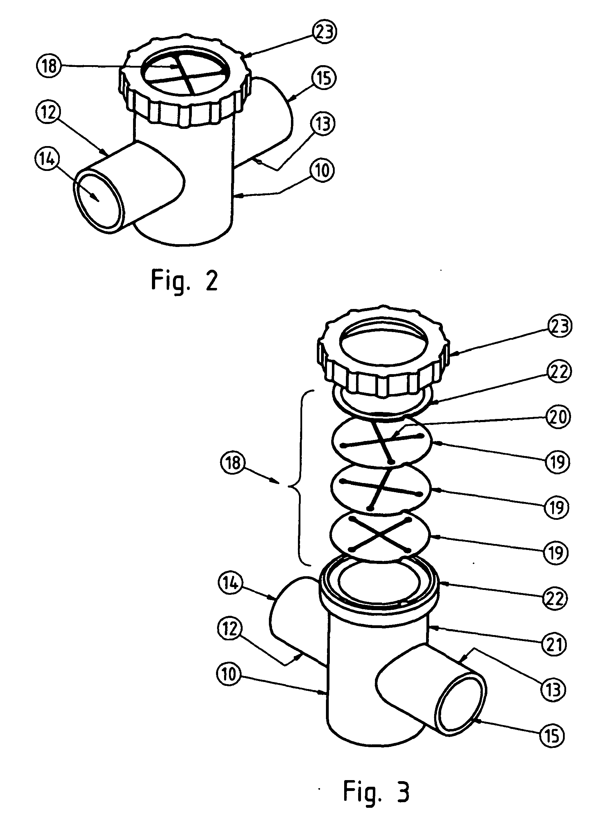

- Fig. 2: eine perspektivische Darstellung eines Zusatzelementes,
- Fig. 3: eine Anordnung wie Fig. 2 mit einer Dichtung der Zuführöffnung in schaubildlicher Darstellung,

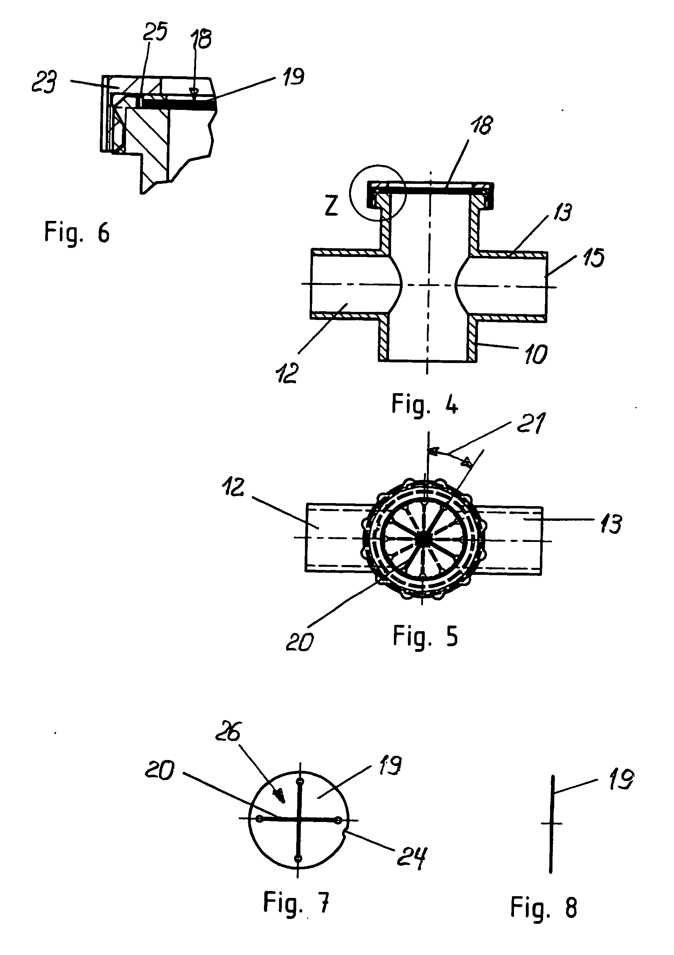

- Fig. 4: eine Schnittdarstellung des Zusatzelementes,
- Fig. 5: eine Draufsicht gemäss Fig. 4,
- Fig. 6: eine vergrösserte Darstellung eines Bereiches Z der Fig. 4 im Bereich der Zuführöffnung,
- Fig. 7: eine Draufsicht auf ein Flächenelement mit einem Kreuzstück,
- Fig. 8: eine Seitenansicht gemäss Fig. 7,
- Fig. 9: ein Zusatzelement mit verbundenen Schlauchelementen für Zuluft- und Abluft und eingesetztem abgedichtetem Instrument und
- Fig. 10: eine vergrösserte Darstellung des Dichtungsbereiches gemäss Einzelheit Y der Fig. 9

Bei der dargestellten Ausbildung ist ein Bronchoskop 1 als Grundelement angeordnet, das über eine Öffnung 2 eines vertikalen Zuführkanals 3 zum Einsetzen eines Instrumentes 4 aufweist und zur Aufnahme eines Zusatzelementes 5 als Adapter dient. In bekannter Weise ist der Zuführkanal 3 mit quer zugeordneten Stutzen 6, 7, 8 und 9 versehen, die für die Hochfrequenzbeatmung sowie als Anschluss für eine Optik, Befeuchtung und Messung ausgebildet sind.

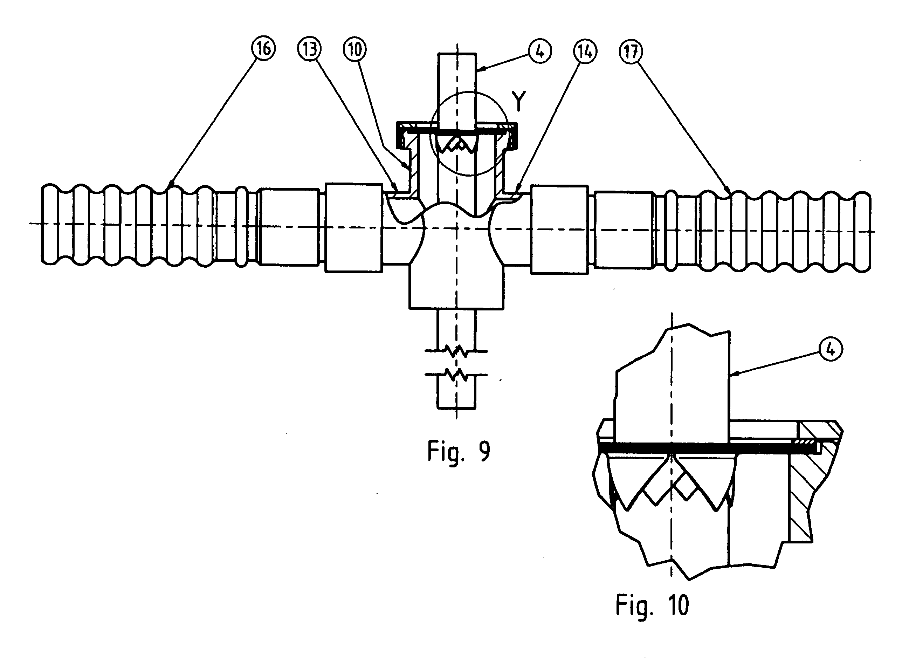

Das auf die Öffnung 2 aufgesetzte Zusatzelement 5 bildet über ein Rohrelement 10 eine Verlängerung für die Instrumentenzuführung 3 des Grundelementes 1, wobei die Instrumente 4 über die obenliegende Zuführöffnung 11 einsetzbar sind. Das Rohrelement 10 besitzt rechtwinklig verbundene Stutzen 12 und 13 mit einer Zuluftöffnung 14 und einer Abluftöffnung 15, die gemäss Fig. 9 mit Verbindungsschläuchen 16, 17 und gegebenenfalls nicht näher dargestellten Einrichtungen zur Luftanfeuchtung und Temperierung im Zuluftbereich sowie zur Reinigung im Abluftbereich geführt sind.

Die Zuführöffnung 11 zum Einsetzen des Instrumentes 4 ist über eine Dichtung 18 ohne ein eingesetztes Instrument 4 verschlossen. Die Dichtung 18 wird durch einzelne ein Dichtungspaket bildende flexible Flächenelemente 19 gebildet, die jeweils mit Kreuzschlitzen 20 versehen sind. Hierbei sind die Kreuzschlitze 20 der übereinander zugeordneten Flächenelemente 19 gegeneinander in einem Winkel 21 versetzt angeordnet.

Das entsprechende Dichtungspaket zur Bildung der Dichtung 18 aus den Flächenelementen 19 wird in einen Aufnahmebereich der Zuführöffnung 11 zwischen beidseitig zugeordnete Scheiben 22 eingesetzt und durch einen aufsetzbaren Gewindering 23 festgelegt.

Die einzelnen Flächenelemente 19 besitzen jeweils versetzte Führungskerben 24, die mit einem Führungssteg 25 im Bereich der Zuführöffnung 11 einsetzbar sind, um einen vorbestimmten Versatz der übereinander liegenden Kreuzschlitze 20 zu gewährleisten.

Beim Einsetzen des Instrumentes 4 in die Zuführöffnung 11 erfolgt über die Kreuzschlitze 20 der Dichtung 18 eine Durchdringung, wobei das eingesetzte Instrument 4 durch die über die Kreuzschlitze gebildeten Klappen 26 der Flächenelemente 19 dichtend umschlossen wird. Hierbei ist eine Anpassung an unterschiedliche Durchmesser des eingesetzten Instruments 4 gegeben.

## Patentansprüche

1. Vorrichtung zur Anvendung in der Hochfrequenzbeatmung mit einem offenen Beatmungssystem, wie bei einem Bronchoskop, Laryngoskop, die vorrictung aufweisend ein Grundelement(1) mit einem düsensystem, und ein zusatzelement (5), wobei über einen Anschluss mittels des Düsensystems aufbereitetes Atemgas mit hohem Druck hochfrequent mit zusätzlich angesaugter Umgebungsluft über eine Öffnung (2) als vertikale Instrumentenzuführung (3) einleitbar ist, **dadurch gekennzeichnet, dass** die Öffnung (2) des Grundelementes (1) mit dem Zusatzelement (5) verbunden ist, das eine Zuluftöffnung (14) für Umgebungsluft und eine Abluftöffnung (15) aufweist sowie in Verlängerung der Instrumentenzuführung (3) eine verschliessbare Zuführöffnung (11) im Zusatzelement (5) für den Instrumenteneinsatz besitzt, wobei die Zuführöffnung (11) durch eine selbst einstellende Dichtung (18) abgeschlossen ist und als flexibles Flächenelement (19) eine durch Schlitze (20) als Kreuzschlitze gebildete Einstecköffnung zur Umfassung einsetzbarer Instrumente (4) mit unterschiedlichen Durchmessern aufweist sowie mehrere Lagen einzelner Flächenelemente (19) der Dichtung (18) parallel angeordnet und die gebildeten Schlitze (20) lagenweise gegeneinander versetzt angeordnet sind.

2. Vorrichtung nach Anspruch 1, **dadurch gekennzeichnet, dass** die lagenweise angeordneten Flächenelemente (19) der Dichtung (18) aus Werkstoffen unterschiedlicher Shorehärte gebildet sind.

3. Vorrichtung nach Anspruch 1 oder 2, **dadurch gekennzeichnet, dass** das Zusatzelement (5) mit seiner Zuführung (10) die zugeordnete Zuluftöffnung (14) für die Umgebungsluft und die Abluftöffnung (15) für die Abluft als Anschlusstutzen (12, 13) aufweist.

4. Vorrichtung nach einem der Ansprüche 1 bis 3, **dadurch gekennzeichnet, dass** die Abluftöffnung (15) über einen Verbindungsschlauch (17) mit einer Reinigungskammer verbunden ist.

5. Vorrichtung nach einem der Ansprüche 1 bis 4, **dadurch gekennzeichnet, dass** die Zuluftöffnung (14) vorgeschaltete Zusatzeinheiten zur Luftanfeuchtung und Temperierung aufweist.

6. Vorrichtung nach einem der Ansprüche 1 bis 5, **dadurch gekennzeichnet, dass** die einsetzbaren Flächenelemente (19) der Dichtung (18) als Dichtungssatz gegeneinander versetzte Kerben (24) aufweisen, die einen Versatz der Schlitze (20) definieren und die Kerben (24) mit einem Führungssteg (25) der Zuführöffnung (11) korrespondieren.

7. Vorrichtung nach einem der Ansprüche 1 bis 6, **dadurch gekennzeichnet, dass** das Zusatzelement (5) als aufsetzbarer Adapter ausgebildet ist.

8. Vorrichtung nach einem der Ansprüche 1 bis 6. **dadurch gekennzeichnet, dass** das Grundelement (1) und Zusatzelement (5) eine Einheit bilden.

## Claims

1. Device for use in high-frequency respiration with an open respiration system, as in a bronchoscope, laryngoscope, the device having a base element (1) having a nozzle system, and an auxiliary element (5), respiratory gas prepared using the nozzle system being able to be introduced via a connection at high pressure and at high frequency with additionally drawn-in ambient air via an opening (2) as a vertical instrument supply (3), **characterised in that** the opening (2) of the base element (1) is connected to the auxiliary element (5) which has an incoming air opening (14) for ambient air and a discharge air opening (15) and, in continuation of the instrument supply (3), has a closable supply opening (11) in the auxiliary element (5) for the use of the instrument, the supply opening (11) being closed by means of a self-adjusting seal (18) and, acting as a flexible surface element (19), having an insertion opening which is formed by slots (20) in the form of transverse slots for surrounding usable instruments (4) having various diameters and a plurality of layers of individual surface elements (19) of the seal (18) being arranged in a parallel manner and the slots (20) formed being arranged so as to be offset with respect to each other in layers.

2. Device according to claim 1, **characterised in that** the surface elements (19) of the seal (18) that are arranged in layers are formed from materials having a different Shore hardness.

3. Device according to claim 1 or claim 2, **characterised in that** the auxiliary element (5) with the supply (10) thereof has the associated supply opening (14) for the ambient air and the discharge air opening (15) for the discharge air as connecting pieces (12, 13).

4. Device according to any one of claims 1 to 3, **characterised in that** the discharge air opening (15) is connected to a cleaning chamber by means of a connection hose (17).

5. Device according to any one of claims 1 to 4, **characterised in that** the incoming air opening (14) has upstream auxiliary units for air humidification and temperature control.

6. Device according to any one of claims 1 to 5, **characterised in that** the usable surface elements (19) of the seal (18) have, as a sealing set, notches (24) which are offset with respect to each other and which define a displacement of the slots (20) and the notches (24) correspond to a guiding web (25) of the supply opening (11).

7. Device according to any one of claims 1 to 6, **characterised in that** the auxiliary element (5) is constructed as an adapter which can be fitted.

8. Device according to any one of claims 1 to 6, **characterised in that** the base element (1) and auxiliary element (5) form a unit.

## Revendications

1. Dispositif destiné à être utilisé pour la ventilation haute fréquence, avec un système de ventilation ouvert, comme pour un bronchoscope, un laryngoscope, le dispositif étant doté d'un élément de base (1) avec un système de buse et d'un élément supplémentaire (5), sachant que, par l'intermédiaire d'un raccord, au moyen du système de buse, un gaz respiratoire traité peut être introduit à haute fréquence, sous pression élevée, avec de l'air ambiant, aspiré en plus, par une ouverture (2), qui est réalisée en tant qu'ouverture d'introduction verticale (3) d'instruments, **caractérisé en ce que** l'ouverture (2) de l'élément de base (1) est reliée à l'élément supplémentaire (5), qui est doté, d'une ouverture de prise d'air (14) pour l'air ambiant et d'une ouverture d'échappement d'air (15), ainsi que d'une ouverture d'admission (11), qui, située en prolongement de l'ouverture d'introduction d'instruments (3), dans l'élément supplémentaire (5), peut être fermée, sachant que l'ouverture d'admission (11) est fermée, par un joint d'étanchéité auto ajustable (18) et est dotée, en tant qu'élément plan, flexible (19), d'une ouverture d'insertion, réalisée sous la forme de fentes en croix (20), pour accueillir des instruments (4) insérables, de différents diamètres, et que plusieurs couches d'éléments plans, individuels (19) du joint d'étanchéité (18) sont disposées parallèlement, et que les fentes formées (20) sont décalées les unes par rapport aux autres, d'une couche à l'autre.

2. Dispositif selon la revendication 1, **caractérisé en ce que** les éléments plans (19) du joint d'étanchéité (18) disposés en couches sont réalisés en matériaux de dureté Shore différente.

3. Dispositif selon revendication 1 ou 2, **caractérisé en ce que** l'élément supplémentaire (5), est doté, avec son corps tubulaire (10), de l'ouverture de prise d'air (14) pour l'air ambiant et de l'ouverture d'échappement (15) pour l'air évacué, en tant que tubulures de raccordement (12, 13).

4. Dispositif selon l'une des revendications 1 à 3, **caractérisé en ce que** l'ouverture d'échappement d'air (15) est reliée à une chambre d'épuration par l'intermédiaire d'un tuyau de liaison (17).

5. Dispositif selon l'une des revendications 1 à 4, **caractérisé en ce que** l'ouverture de prise d'air (14) est dotée d'unités supplémentaires, connectées en amont, pour l'humidification de l'air et la mise à température.

6. Dispositif selon l'une des revendications 1 à 5, **caractérisé en ce que** les éléments plats (19) du joint d'étanchéité (18), qui peuvent être insérés en tant que jeu de joint, sont dotés d'encoches (24), qui, décalées les unes par rapport aux autres, définissent un décalage des fentes (20), et que les encoches (24) correspondent à une saillie de guidage (25) de l'ouverture d'admission (11).

7. Dispositif selon l'une des revendications 1 à 6, **caractérisé en ce que** l'élément supplémentaire (5) est réalisé sous la forme d'un adaptateur.

8. Dispositif selon l'une des revendications 1 à 6, **caractérisé en ce que** l'élément de base (1) et l'élément supplémentaire (5) forment une unité.
